(19) Europäisches Patentamt
European Patent Office
Office européen des brevets

(11) EP 0 835 112 B1

(12) **EUROPÄISCHE PATENTSCHRIFT**

(45) Veröffentlichungstag und Bekanntmachung des Hinweises auf die Patenterteilung:
**10.09.2003 Patentblatt 2003/37**

(21) Anmeldenummer: **97918095.7**

(22) Anmeldetag: **01.04.1997**

(51) Int Cl.[7]: **A61K 31/555**

(86) Internationale Anmeldenummer:
**PCT/EP97/01643**

(87) Internationale Veröffentlichungsnummer:
**WO 97/036595 (09.10.1997 Gazette 1997/43)**

(54) **ARZNEIMITTELZUBEREITUNGEN ENTHALTEND TUMORHEMMEND WIRKENDE RUTHENIUM(III)-KOMPLEXE**

MEDICAMENT PREPARATIONS CONTAINING TUMOUR-INHIBITING RUTHENIUM (III) COMPLEXES

PREPARATIONS MEDICAMENTEUSES CONTENANT DES COMPLEXES DE RUTHENIUM (III) A ACTION ANTITUMORALE

(84) Benannte Vertragsstaaten:
**AT BE CH DE DK ES FI FR GB GR IE IT LI LU NL PT SE**

(30) Priorität: **28.03.1996 DE 19612291**

(43) Veröffentlichungstag der Anmeldung:
**15.04.1998 Patentblatt 1998/16**

(73) Patentinhaber: **Faustus Forschungs Cie. Translational Cancer Research GmbH
04109 Leipzig (DE)**

(72) Erfinder: **Keppler, Bernhard K., Prof. Dr.Dr.
2191 Gaweinstal (AT)**

(74) Vertreter: **Grünecker, Kinkeldey, Stockmair & Schwanhäusser Anwaltssozietät
Maximilianstrasse 58
80538 München (DE)**

(56) Entgegenhaltungen:


- **B.K. KEPPLER ET AL.: "Synthesis, molecular structure, and tumor-inhibiting properties of imidazolium trans-bis(imidazole)tetrachlororuthenate(I II) and its methyl substituted derivatives." INORG. CHEM., Bd. 26, Nr. 26, 1987, Seiten 4366-4370, XP002040469 in der Anmeldung erwähnt**
- **C. ANDERSON ET AL.: "Synthesis of the tetrachlorobis(5-nitroimidazole)ruthenium( III) anion and solution chemistry in water and methanol." INORG. CHEM. ACTA, Bd. 233, Nr. 1-2, 1995, Seiten 33-41, XP002040470**
- **R. GAGLIARDI ET AL.: "Antimetastatic action and toxicity on healthy tissues of Na(trans-RuCl4(DMSO)Im) in the mouse." CLIN. EXP. METASTASIS, Bd. 12, Nr. 2, 1994, Seiten 93-100, XP002040471**


Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 835 112 B1

Printed by Jouve, 75001 PARIS (FR)

## Beschreibung

Technisches Gebiet

Die Erfindung bezieht sich auf Arzneimittelzubereitungen enthaltend tumorhemmend wirkende Ruthenium (III)-Komplexe und deren verwendung zur herstellung von arzneimittelzubereitungen zur behandlung von krebskrankheiten.

Stand der Technik

Komplexverbindungen der allgemeinen Formel

$$(BH_n)_m[RuX_{3+nm-q-r}(OH)_r(BH_p)_{3-nm+q}]_{1+p+q}$$

worin

- B einen ein- oder mehrkernigen, einen oder mehrere Stickstoffatome enthaltenden basischen Heterocyclus,
- X Chlor oder Brom,
- m 1 oder 2,
- n 1 oder 2, wobei die Summe aus n und m nicht größer als 3 ist,
- p 0 oder 1, aber nicht 1, falls n=1 ist,
- q 0 oder 1, aber nicht 1, falls p=1 ist und
- r 0 oder 1

bedeuten, zeigen eine vorteilhafte tumorhemmende Wirksamkeit bei günstiger Toxizität. [Patent EP 85904433.1-2110].

Von besonderer Bedeutung sind die Komplexe des Rutheniums mit Pyrazol und Imidazol, die z.B. durch F. Kralik et al., *Collection Czechoslov. Chem. Commun*. **26** (1961) 1298, oder B.K. Keppler et al., *Inorg. Chem*., **26** (1987) 4366-4370, untersucht wurden, aber auch die Komplexe des Rutheniums mit Indazol und Dimethylsulfoxid [B.K. Keppler et al., *Anticancer Res*., **9** (1989) 761-766), G. Mestroni et al., *J. Am. Chem. Soc*. **111** (1989) 7068-7071, G. Mestroni et al., *Inorg. Chem*., **34** (1995) 4722-4734.]

Ein großer Nachteil der bisher eingesetzten Rutheniumkomplexe ist deren durch das heterocyclische Kation ($BH^+$) bedingte geringe Wasserlöslichkeit.

Darstellung der Erfindung

Überraschend wurde nun gefunden, daß Komplexverbindungen der allgemeinen Formel I

$$\{G\}^{-(n+p+2r(p-1)-3)}\{[RuX_{6-n-p-q-2r}B_n(H_2O)_p(OH)_q(O)_r]_{2r+1}\}^{n+p+2r(p-1)-3} \qquad \mathbf{I}$$

wobei

$$n+p+2r(p-1)-3q \neq 0$$

- G ein geeignetes Gegenion, jedoch kein geladener Heterocyclus,
- B ein ein- oder mehrkerniger basischer Heterocyclus mit einem oder mehreren Stickstoffatomen,
- X Halogenide, Pseudohalogenide, $HCO_3$-, RCOO- mit R=Alkyl, Alkenyl, mit 1 bis 6 C-Atomen (substituiert u. unsubstituiert), Aryl (substituiert u. unsubstituiert),
- n 1, 2 oder 3, jedoch $n+p \neq 3$,
- p 0 oder 1 bzw. 0 oder 0.5p falls r=0.5,

q 0 oder 1 bzw. 0 oder 0.5q falls r=0.5, $\Sigma$ n, p, q$\leq$6 und
r 0 oder 0.5 ist,

und besonders die Komplexe der allgemeinen Formel II

$$M_{3-n-p-2pr}[RuX_{6-n-p-q-2r}B_n(H_2O)_p(OH)_q(O)_r]_{2r+1} \qquad \mathbf{II}$$

wobei

M ein Alkalimetallkation oder Ammonium,
B ein ein- oder mehrkerniger basischer Heterocyclus mit einem oder mehreren Stickstoffatomen,
X Halogen, bevorzugt Chlorid oder Bromid,
n 1 oder 2,
p 0 oder 1 bzw. 0 oder 0.5 falls r=0.5,
q 0 oder 1 bzw. 0 oder 0.5 falls r=0.5 und
r 0 oder 0.5 ist,

bei nahezu identischen therapeutischen Eigenschaften und sehr ähnlichem Verhalten gegenüber Wasser gut wasserlöslich sind.

Besonders bevorzugt sind Arzneimittelzubereitungen enthaltend Komplexsalze der allgemeinen Formel II'

$$M'_{3-n-p-2pr}[RuX_{6-n-p-q-2r}B'_n(H_2O)_p(OH)_q(O)_r]_{2r+r} \qquad \mathbf{II'}$$

wobei

M' Lithium, Natrium, Kalium und Ammonium,
B' 1*H*-Indazol oder 1*H*-Imidazol und deren Derivate,
X Chlor,
p 0 oder 1 bzw. 0 oder 0.5 falls r=0.5, jedoch 0 wenn q$\neq$0,
q 0 oder 1 bzw. 0 oder 0.5 falls r=0.5, jedoch 0 wenn p$\neq$0,
n und r die vorstehende Bedeutung haben.

B':

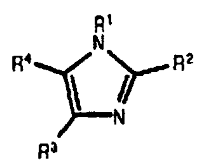

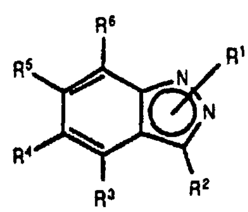

$R^1$ ..... $R^6$=H, Alkyl, Alkenyl u. Aryl, mit 1 bis 6 C-Atomen, substituiert u. unsubstituiert

Ganz besonders bevorzugt sind Arzneimittelzubereitungen enthaltend Komplexsalze der allgemeinen Formel II"

$$M''_{3-n-p-2pr}[RuX_{6-n-p-q-2r}B'_n(H_2O)_p(OH)_q(O)_r]_{2r+r} \qquad \mathbf{II''}$$

wobei
M" Natrium, B', X, n, p, q und r die vorstehende Bedeutung haben.

Gegenstand der Erfindung sind daher Arzneimittelzubereitungen enthaltend diese Rutheniumkomplexsalze, deren Verwendung zur Herstellung von Arzneimittelzubereitungen zur behandlung von Krebskrankheiten.

Ganz besonders bevorzugt sind Arzneimittelzubereitungen enthaltend *trans*-Natrium-tetrachlorobis(imidazol) ruthenat, *trans*-$Na[Ru^{III}Cl_4(im)_2]$ und *trans*-Natrium-tetrachlorobis(indazol)ruthenat(III), *trans*-$Na[Ru^{III}Cl_4(ind)_2]$. Die

Verbindungen sind neu und daher ebenfalls Gegenstand der Erfindung.

Die Herstellung der Natriumsalze der Rutheniumkomplexe II" ist ebenfalls Gegenstand der Erfindung.

Die erfindungsgemäßen Arzneimittelzubereitungen werden vor allem intravenös, aber auch intramuskulär, intraperitoneal, subkutan oder peroral verabreicht. Auch eine äußerliche Applikation ist möglich. Bevorzugt ist die Verabreichung durch intravenöse Injektion oder intravenöse Infusion.

Die Arzneimittelzubereitungen werden nach an sich bekannten Verfahren hergestellt, wobei die erfindungsgemäßen Verbindungen als solche oder gegebenenfalls in Kombination mit geeigneten pharmazeutischen Trägerstoffen eingesetzt werden. Enthalten die neuen pharmazeutischen Zubereitungen neben dem Wirkstoff pharmazeutische Trägerstoffe, beträgt der Wirkstoffgehalt dieser Mischungen 0.1 bis 99.5, vorzugsweise 0.5 bis 95 Gew.% der Gesamtmischung.

In Übereinstimmung mit der Erfindung wird ein Wirkstoff in jeder geeigneten Formulierung angewandt unter der Voraussetzung, daß die Ausbildung bzw. Aufrechterhaltung von ausreichenden Wirkstoffpegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für Applikationen z.B. am Menschen bestimmt sind, etwa 0.1 bis 500 mg vorteilhafterweise 10 bis 200 mg und insbesondere 50 bis 150 mg Wirkstoff enthalten.

Im allgemeinen werden in der Humanmedizin der oder die Wirkstoffe bei oraler Gabe in einer Tagesdosis von 0.1 bis 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0.1 bis 5, vorzugsweise 1 bis 3 mg/kg Körpergewicht. Bei einer oralen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter der zu behandelnden Individuen, der Art und Schwere der Erkrankung, der Art der Zubereitung und der Applikation der Arzneimittelzubereitungen sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Es kann sich auch als zweckmäßig erweisen, die Arzneimittelzubereitungen nur einmalig oder im Abstand von mehreren Tagen zu verabreichen.

Die Festlegung der jeweils erforderlichen optimalen Dosierung und Applikationsart der Wirkstoffe kann durch jeden Fachmann aufgrund seines Fachwissens erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption der Arzneimittelzubereitung im Gastrointestinaltrakt bewirkt, so daß z.B. eine bessere Verträglichkeit, Protahierung oder Retardierung erreicht wird. Gelatinekapseln können den Arzneistoff vermischt mit einem festen, z.B. Calciumcarbonat oder Kaolin, oder einem öligen, z.B. Oliven-, Erdnuß-, oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycatanol, Polyoxyethylensorbitolmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können eine Rutheniumkomplexverbindung in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuß-, oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmittel enthalten.

Wäßrige Lösungen, die gegebenenfalls kurzfristig zubereitet werden, können Konservierungsmittel, z.B. Methyl- oder Propylhydroxybenzoate; Verdikkungsmittel; Geschmacksmittel; Süßungsmittel, z.B. Saccharose, Lactose, Natriumcyclamat, Dextrose, Invertzuckersirup, sowie Geschmacksmittel und Farbstoffe enthalten.

Zur parenteralen Anwendung der Arzneistoffe dienen steril injizierbare, gegebenenfalls kurzfristig herzustellende, wäßrige Lösungen, isotonische Salzlösungen oder sonstige Lösungen.

Die folgenden Beispiele erläutern die Erfindung näher, ohne sie einzuschränken.

Herstellungsbeispiele

Allgemeine Vorschrift

Die Herstellung der Natriumsalze der Rutheniumkomplexe II" erfolgt bevorzugt über die Komplexsalze III.

$$(B'H)_{3-n-p-2pr}[RuX_{6-n-p-q-2r}B'_n(H_2O)_p(OH)_q(O)_r]_{2r+1} \quad \textbf{III}$$

mit der obigen Bedeutung von B'.

III wird bevorzugt nach L.H. Hurley et. al., *Trends Pharmacol. Sci.*, **9** (1988) 402-407, und K.-G. Lipponer, *Dissertation* (1992), Universität Heidelberg, S. 1-116 u. 173-196, hergestellt. Durch Umsetzung mit Tetraphenylphosphoniumiodid erhält man zunächst das Tetraphenylphosphonium-Salz. Mit Natriumtetraphenylborat kann schließlich das Tetraphenylphosphoniumkation gegen das Natriumkation ausgetauscht werden.

Detail-Vorschriften

***Trans*-tetraphenylphosphoniumtetrachlorobis(imidazol)ruthenat(III), *trans*-$Ph_4P[Ru^{III}Cl_4(im)_2]$**

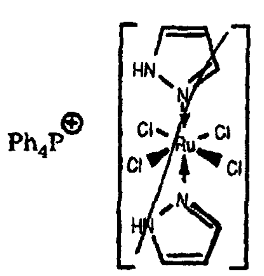

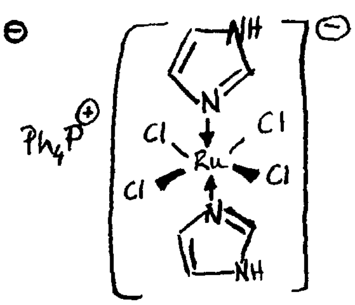

| | | |
|---|---|---|
| Ansatz: | 22.5 mg *trans*-HIm[$RuCl_4(im)_2$] | (0.5 mmol) |
| | 245 mg Tetraphenylphosphoniumiodid | (0.52 mmol) |
| | 270 ml Methanol | |

Zu einer Lösung von 225 mg (0.5 mmol) *Trans*-imidazolium-tetrachlorobis(imidazol)ruthenat(III), *trans*-HIm [$Ru^{III}Cl_4(im)_2$], in 270 ml Methanol werden 245 mg (0.52 mmol) Tetraphenylphosphoniumiodid fest zugegeben. Nach wenigen Sekunden beginnt eine Niederschlagsbildung. Die Suspension wird 10 min gerührt und die Präzipitation während weiterer 30 min bei -18°C vervollständigt. Der Niederschlag wird danach abgesaugt, zuerst mit 50 ml Methanol und danach 4 mal mit 40 ml Dichlormethan gewaschen. Die Substanz wird mehrere Tage bei 80°C im Hochvakuum getrocknet.

Ausbeute: 322 mg (90%) blaßorange hygroskopische Mikrokristalle; Zers.P.: 308°C.

| Elementaranalyse: $C_{30}H_{28}Cl_4N_4PRu$ / $M_r$ = 718.43 | | | | | | |
|---|---|---|---|---|---|---|
| ber. | C: 50.15 | H: 3.93 | Cl: 19.74 | N: 7.80 | P: 4.31 | Ru: 14.07 |
| gef. | C: 49.81 | H: 4.03 | Cl: 19.64 | N: 7.85 | P: 7.85 | Ru: - |

$^1$H-NMR (DMSO($d_6$)) δ: 7.79 (s, 8H, *H*-ph.), 7.73 (s, 4H, *H*-ph.), 7.69 (s, 4H, *H*-ph.), -2.8 (s, b, 2H, N*H*), -7.9 (s, b, 2H, 2-*H*).
IR, (4400 - 400 $cm^{-1}$, KBr) ν, $cm^{-1}$: 3436 b, 3201 s, 1442 vs, 1110 vs, 1060 vs, 755 s, 724 vs, 692 s, 533 s.
UV, (250 - 800 nm, $H_2O/CH_3CN$ (50/50)) $\lambda_{max}$, nm (ε, $1*mol^{-1}*cm^{-1}$): 268 nm (5.4 * $10^3$), 275 nm (4.6 * $10^3$), 355 nm (3.3 * $10^3$), 403 nm (0.6 * $10^3$).

***Trans*-tetraphenylphosphonium-tetrachlorobis(indazol)ruthenat(III), *trans*-$Ph_4P[Ru^{III}Cl_4(ind)_2]$**

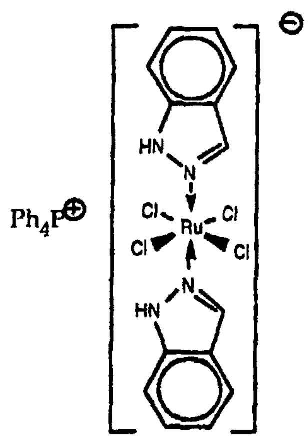

| | | |
|---|---|---|
| Ansatz: | 149 mg *trans*-HInd[$RuCl_4(ind)_2$] | (0.25 mmol) |
| | 188 mg Tetraphenylphosphoniumchlorid | (0.50 mmol) |
| | 500 ml Wasser | |

149 mg *Trans*-indazolium-tetrachlorobis(indazol)ruthenat(III), *trans*-HInd[$Ru^{III}Cl_4(ind)_2$], werden über 1 h in 500 ml $H_2O$ gelöst. Es bildet sich über der orangefarbenen Lösung ein Film aus noch ungelösten Bestandteilen aus. 188 mg Tetraphenylphosphoniumchlorid (0.5 mmol) werden als Feststoff zur Lösung hinzugegeben. Die Präzipitation beginnt kurz darauf, über Nacht wird weiter gerührt. Der Niederschlag wird abgesaugt und das noch inhomogene Präzipitat mehrfach mit $H_2O$ gewaschen, um Reste von $Ph_4PCl$ zu entfernen. Danach wird das Produkt in 35 ml Aceton

gelöst und das Produkt von noch vorhandenem *trans*-HInd[$RuCl_4(ind)_2$] durch Fällung mit 140 ml Diethylether abgetrennt. Der orangefarbene Überstand wird verworfen. Das Produkt wird zuerst mit 50 ml Diethylether /Aceton (4:1), danach je 50 ml im Verhältnis 3:1, 2:1 und 30 ml im Verhältnis 1:1 (kalt) gewaschen. Die Substanz wird mehrere Tage im Hochvakuum bei 80°C getrocknet.

Ausbeute: 152 mg (74%), quitte gelbes Kristallpulver, hygroskopisch, Zers.P.: 238°C.

| Elementaranalyse: $C_{38}H_{32}Cl_4N_4PRu$ / $M_r$ = 818.56 | | | | | | |
|---|---|---|---|---|---|---|
| ber. | C: 55.76 | H: 3.94 | Cl: 17.33 | N: 6.84 | P: 3.78 | Ru: 12.35 |
| gef. | C: 55.42 | H: 3.97 | Cl: 17.58 | N: 6.77 | P: 3.86 | Ru: - |

$^1$H-NMR, (DMSO(d6)) δ: 7.94 (s, b, 2H, *H*-ph.), 7.77 (s, 12H, *H*-ph.), 7.70 (s, 6H, *H*-ph.), 4.38 (s, b, 2H, 5-*H*-Ind, 6-*H*-Ind), 3.22 (m, 2H, 4-*H*-Ind, 5-*H*-Ind).
IR, (4400 - 400 $cm^{-1}$, KBr) (ν, $cm^{-1}$: 3312 b, 3058 m, 1625 s, 1438 vs, 1355 s, 1244 m, 1108 vs, 998 m, 959 m, 755 s, 724 vs, 690 s, 665 s, 528 vs.
UV, (250 - 800 nm, $CH_3CN$) ($\lambda_{max}$, nm (ε, 1 * $mol^{-1}$ * $cm^{-1}$): 268 (14 * $10^3$), 276 (15 * $10^3$), 291 (16 * $10^3$), 304 (17 * $10^3$), 383 (5.4 * $10^3$), 445 (0.6 * $10^3$).

***Trans*-Natrium-tetrachlorobis(imidazol)ruthenat(III), *trans*-Na[$Ru^{III}Cl_4(im)_2$]**

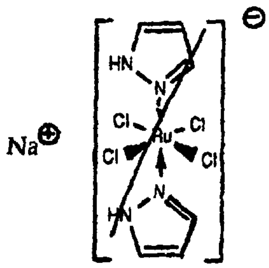

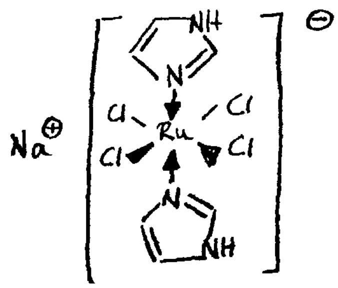

| | | |
|---|---|---|
| Ansatz: | 359 mg *trans*-HIm[$RuCl_4(im)_2$] | (0.50 mmol) |
| | 205 mg Natriumtetraphenylborat | (0.60 mmol) |
| | 60 ml Methanol | |

In 60 ml Methanol (kalt) wird Natriumtetraphenylborat (Na[$(C_6H_5)_4$B], 205 mg, 0.60 mmol) gelöst und direkt danach *Trans*-tetraphenylphosphoniumtetrachlorobis(imidazol)ruthenat(III), *trans*-$Ph_4P[Ru^{III}Cl_4(im)_2]$, als Feststoff hinzugegeben. Die gelblich-weiße Suspension wird 5 min gerührt und die *Um*fällung unter Hinzunahme eines Ultraschallbads beschleunigt. Man läßt den neu gebildeten weißen Niederschlag aus Tetraphenylphosphoniumtetraphenylborat kurz sich absetzen und filtriert die orangefarbene Lösung über einen Glasfiltertiegel ab. Dabei tropft die methanolische Lösung direkt in 300 ml Diethylether als Vorlage. Die erneute Präzipitation vervollständigt man 30 min bei -18°C und filtriert das orangefarbene Produkt, das anschließend mit 10 ml Diethylether, 2 x 10 ml Dichlormethan und 5 ml eisgekühltem Aceton gewaschen wird, ab. Durch Zugabe von wenigen ml eisgekühltem Methanol auf den Glasfiltertiegel wird das Produkt gelöst und tropft zur Umfällung erneut in 250 ml Diethylether als Vorlage. Die erneute Präzipitation aus einer milchigen Lösung wird bei -18°C vervollständigt. Das ockerfarbene Produkt wird auf einer Mikrofilternutsche gesammelt und erneut mit Dichlormethan und wenig eiskaltem Aceton gewaschen. Es ist wichtig, daß das Lösen in Methanol jeweils so kurz wie möglich erfolgt, da sich unter Zersetzung des Komplexanions [$RuCl_3(im)_2$ (MeOH)) (orangefarben) bildet. Das Produkt wird mehrere Tage bei 80°C im Hochvakuum getrocknet.

Ausbeute: 119 mg (58%) eines ockerfarbenen hygroskopischen Feststoffes, Zers.-P.: > 280°C.

| Elementaranalyse: $C_6H_8Cl_4N_4NaRu$ * 0.5 $H_2O$ / $M_r$ = 411.04 | | | | | | | |
|---|---|---|---|---|---|---|---|
| ber. | C: 17.53 | H: 2.21 | Cl: 34.50 | N: 13.63 | Na: 5.59 | O: 1.95 | Ru: 24.59 |

(fortgesetzt)

| Elementaranalyse: $C_6H_8Cl_4N_4NaRu$ * 0.5 $H_2O$ / $M_r$ = 411.04 | | | | | | | |
|---|---|---|---|---|---|---|---|
| gef. | C: 17.75 | H: 2.30 | Cl: 34.11 | N: 13.31 | Na: - | O: - | Ru: - |

$^1$H-NMR, (DMSO($d_6$)) δ: -3.1 (s, b, 2H, N*H*), -8.1 (s, b, 2H, 2-*H*); ($D_2O$) δ: -5.3 (s, 2H, 4-*H*), -14.9 (s, b, 5-*H*), -20.2 (s, b, 2-*H*).
IR, (4400 - 400 $cm^{-1}$, KBr) ν, $cm^{-1}$: 3380 s, 3155 m, 1629 b, 1096 s, 1061 vs, 758 b, 656 s, 608 s.
UV, (200 - 800 nm, $H_2O$) $\lambda_{max}$, mit (ε, 1 * $mol^{-1}$ * $cm^{-1}$): 235 (11 * $10^3$), 349 (3.1 * $10^3$), 396 (0.5 * $10^3$).

***Trans*-Natrium-tetrachlorobis(indazol)ruthenat(III), *trans*-Na[$Ru^{III}Cl_4(ind)_2$]**

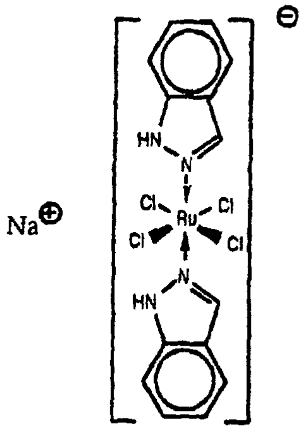

| Ansatz: | 224 mg *trans*-$Ph_4P[RuCl_4(ind)_2]$ | (0.275 mmol) |
|---|---|---|
| | 113 mg Natriumtetraphenylborat | (0.33 mmol) |
| | 10 ml Methanol | |

Natriumtetraphenylborat (Na[$(C_6H_5)_4$B], 113 mg, 0.33 mmol) wird in 10 ml Methanol gelöst. 224 mg (0.275 mmol) *Trans*-tetraphenylphosphoniumtetrachlorobis(indazol)ruthenat(III), *trans*-$Ph_4P[Ru^{III}Cl_4(ind)_2]$, werden fest hinzugegeben und 5 min bei Raumtemperatur gerührt. Es bildet sich ein weißer Niederschlag aus Tetraphenylphosphoniumtetraphenylborat, der kurz darauf über eine Mikrofilternutsche abgetrennt wird. Das Filtrat läßt man dabei direkt in eine Vorlage aus 25 ml Diethylether und 100 ml Petrolether 40/60 tropfen. Die dabei beginnende Fällungsreaktion, bei der das Produkt karmesinrot ausfällt, vervollständigt man durch Zugabe von weiteren 150 ml Petrolether. Die Präzipitation wird über 30 min bei -18°C vervollständigt und die ausgefallenen roten Flocken mit einer Glaspipette vom Kolbenboden abgezogen. In einer Mikrofilternutsche (Prittengröße 3, Ø 1 cm) wird das Produkt gesammelt und zuerst mit 10 ml Petrolether und anschließend mit reichlich Dichlormethan gewaschen. Die Trocknung erfolgt über mehrere Tage bei 80°C am Hochvakuum.
Ausbeute: 116 mg (84%) eines dunkelbraunen hygroskopischen Pulvers, Zers.-P.: > 290°C.

| Elementaranalyse: $C_{14}H_{12}Cl_4N_4NaRu$ / $M_r$ = 502.15 | | | | | | |
|---|---|---|---|---|---|---|
| ber.<br>gef. | C: 33.48<br>C: 33.36 | H: 2.41<br>H: 2.55 | Cl: 28.24<br>Cl: 28.11 | N: 11.15<br>N: 11.02 | Na: 4.59<br>Na: - | Ru: 20.13<br>Ru: - |

$^1$H-NMR, (DMSO($d_6$)) δ: 4.33 (s, b, 2H, ), 3.14 (s, 3H, ); ($D_2O$) d: 3.23 (s, b, 3H, 5-*H*), 2.68 (s, b, 2H, 6-*H*), 2.31 (s, b, 3H, 4-*H*), 1.49 (s, b, 2H, 7-*H*).
IR, (4400 - 400 $cm^{-1}$, KBr) ν, $cm^{-1}$: 3342 vs, 1626 vs, 1509 m, 1356 vs, 1241 s, 1084 s, 964 m, 745 vs, 658 s, 435 w.

UV, (200 - 800 nm, $H_2O$) $\lambda_{max}$, nm ($\varepsilon$, 1 * $mol^{-1}$ * $cm^{-1}$): 237 (17 * $10^3$), 287 (20 * $10^3$), 357 (4.4 * $10^3$), 419 (1.2* $10^3$).

**Patentansprüche**

**1.** Arzneimittelzubereitungen, enthaltend eine oder mehrere Komplexverbindungen der allgemeinen Formel I

$$\{G\}^{-(n+p+2r(p-1)-3)}\{[RuX_{6-n-p-q-2r}B_n(H_2O)_p(OH)_q(O)_r]_{2r+1}\}^{n+p+2r(p-1)-3} \qquad (I)$$

wobei

$$n+p+2r(p-1)-3q \neq 0$$

- G ein geeignetes Gegenion, jedoch kein geladener Heterocyclus,
- B ein ein- oder mehrkerniger basischer Heterocyclus mit einem oder mehreren Stickstoffatomen,
- X Halogenide, Pseudohalogenide, $HCO_3^-$, $RCOO^-$ mit R=Alkyl, Alkenyl mit 1 bis 6 C-Atomen, (substituiert u. unsubstituiert), Aryl (substituiert u. unsubstituiert),
- n 1, 2 oder 3, jedoch $n+p \neq 3$
- p 0 oder 1 bzw. 0 oder 0.5p falls r=0.5,
- q 0 oder 1 bzw. 0 oder 0.5q falls r=0.5, $\Sigma$ n, p, q $\leq 6$ und
- r 0 oder 0.5 ist,

bedeuten.

**2.** Arzneimittelzubereitungen, enthaltend eine oder mehrere Komplexverbindungen der allgemeinen Formel II

$$M_{3-n-p-2pr}[RuX_{6-n-p-q-2r}B_n(H_2O)_p(OH)_q(O)_r]_{2r+1} \qquad (II)$$

wobei

- M ein Alkalimetallkation oder Ammonium,
- B ein ein- oder mehrkemiger basischer Heterocyclus mit einem oder mehreren Stickstoffatomen,
- X Halogen, bevorzugt Chlorid oder Bromid,
- n 1 oder 2,
- p 0 oder 1 bzw. 0 oder 0.5 falls r=0.5,
- q 0 oder 1 bzw. 0 oder 0.5 falls r=0.5 und
- r 0 oder 0.5 ist

**3.** Arzneimittelzubereitungen, enthaltend eine oder mehrere Komplexverbindungen der allgemeinen Formel II'

$$M'_{3-n-p-2pr}[RuX_{6-n-p-q-2r}B'_n(H_2O)_p(OH)_q(O)_r]_{2r+r} \qquad (II')$$

- M' Lithium, Natrium und Kalium,
- B' 1 *H*-indazol oder 1*H*-Imidazol und deren Derivate,
- X Chlor,
- p 0 oder 1 bzw. 0 oder 0.5 falls r=0.5, jedoch 0 wenn $q \neq 0$
- q 0 oder 1 bzw. 0 oder 0.5 falls r=0.5, jedoch 0 wenn $p \neq 0$,
- n und r die vorstehende Bedeutung haben.

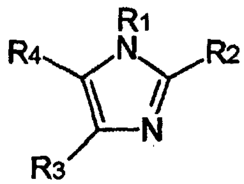
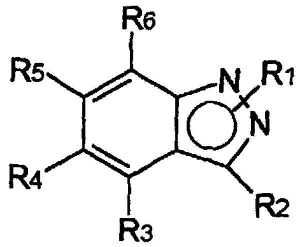

$R^1$ ... $R^6$ = H, Alkyl, Alkenyl u. Aryl, mit 1 bis 6 C-Atomen, substituiert u. unsubstituiert.

**4.** Arzneimittelzubereitungen enthaltend eine oder mehrere Komplexverbindungen der allgemeinen Formel II"

$$M''_{3-n-p-2pr}[RuX_{6-n-p-q-2r}B'_n(H_2O)_p(OH)_q(O)_r]_{2r+r} \quad (II'')$$

wobei
M" Natrium, B', X, n, p, q und r die vorstehende Bedeutung haben.

**5.** Arzneimittelzubereitungen, enthaltend *Trans*-Natrium-tetrachlorobis(imidazol)ruthenat(III) oder *Trans*-Natriumtetrachlorobis(indazol)ruthenat(III).

**6.** Arzneimittelzubereitungen, umfassend einen pharmazeutisch annehmbaren Trägerstoff und 0.1 bis 99.5 Gew.% mindestens eine der in einem der Ansprüche 1 bis 5 genannten Komplexverbindung der Formeln (I), (II), (II') oder (II").

**7.** Verfahren zur Herstellung der in den Ansprüchen 2, 3 oder 4 genannten Komplexverbindungen der Formel (II), (II') oder (II"), **dadurch gekennzeichnet, dass** (BH) bzw. (B'H) in den Formeln $(BH)_{3-n-p-2pr}[RuX_{6-n-p-q-2r}B_n(H_2O)_p(OH)_q(O)_r]_{2r+1}$ bzw. $(B'H)_{3-n-p-2pr}[RuX_{6-n-p-q-2r}B'_n(H_2O)_p(OH)_q(O)_r]_{2r+r}$ durch M, M' bzw. M" ausgetauscht werden, wobei B, B', M, M', M" die in den Ansprüchen 2, 3 oder 4 angegebenen Bedeutungen haben.

**8.** Sterile wässrige Lösungen, enthaltend eine oder mehrere der in einem der Ansprüche 1 bis 5 genannten Komplexverbindungen der Formeln (1), (II), (II') oder (II").

**9.** Verwendung einer der in einem der Ansprüche 1 bis 5 genannten Komplexverbindungen der Formeln (I), (II), (II') oder (II") zur Herstellung von Arzneimittelzubereitungen zur Behandlung von Krebskrankheiten.

**Claims**

**1.** Medicament formulations containing one or more complex compounds of the general formula I

$$\{G\}^{-(n+p+2r(p-1)-3)}\{[RuX_{6-n-p-q-2r}B_n(H_2O)_p(OH)_q(O)_r]_{2r+1}\}^{n+p+2r(p-1)-3} \quad (I)$$

wherein

$$n+p+2r(p-1)-3q \neq 0$$

G denotes a suitable counter-ion, but not a charged heterocycle,
B denotes a mononuclear or polynuclear basic heterocycle having one or more nitrogen atoms,
X denotes halides, pseudo-halides, $HCO_3^-$, $RCOO^-$ where R = alkyl, alkenyl having 1 to 6 C atoms, (substituted and unsubstituted), aryl (substituted and unsubstituted),
n denotes 1, 2 or 3, but n+p ≠ 3

p denotes 0 or 1 or 0 or 0.5p if r = 0.5,
q denotes 0 or 1 or 0 or 0.5q if r = 0.5, $\Sigma n, p, q \leq 6$ and
r is 0 or 0.5.

**2.** Medicament formulations containing one or more complex compounds of the general formula II

$$M_{3-n-p-2pr}[RuX_{3-n-p-q-2r}B_n(H_2O)_p(OH)_q(O)_r]_{2r+1} \quad (II)$$

wherein

M is an alkali metal cation or ammonium,
B is a mononuclear or polynuclear basic heterocycle having one or more nitrogen atoms,
X is halogen, preferably chloride or bromide,
n is 1 or 2,
p is 0 or 1 or 0 or 0.5 if r = 0.5,
q is 0 or 1 or 0 or 0.5 if r = 0.5 and
r is 0 or 0.5.

**3.** Medicament formulations containing one or more complex compounds of the general formula II'

$$M'_{3-n-p-2pr}[RuX_{3-n-p-q-2r}B'_n(H_2O)_p(OH)_q(O)_r]_{2r+r} \quad (II')$$

M' lithium, sodium and potassium,
B' 1H-indazole or 1H-imidazole and derivatives thereof,
X chlorine,
p 0 or 1 or 0 or 0.5 if r = 0.5, but 0 when $q \neq 0$
q 0 or 1 or 0 or 0.5 if r = 0.5, but 0 when p # 0,
n and r have the above meaning.

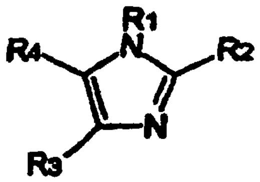

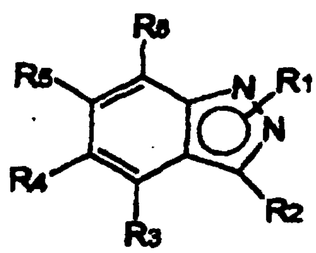

$R^1$... $R^6$ = H, alkyl, alkenyl and aryl, having 1 to 6 C atoms, substituted and unsubstituted.

**4.** Medicament formulations containing one or more complex compounds of the general formula II"

$$M''_{3-n-p-2pr}[RuX_{3-n-p-q-2r}B'_n(H_2O)_p(OH)_q(O)_r]_{2r+r} \quad (II'')$$

wherein
M" sodium, B', X, n, p, q and r have the above meaning.

**5.** Medicament formulations containing *trans*-sodium-tetrachlorobis(imidazole) ruthenate (III) or *trans*-sodium-tetrachlorobis(indazole) ruthenate (III).

**6.** Medicament formulations comprising a pharmaceutically acceptable excipient and 0.1 to 99.5 wt.% of at least one of the complex compound of formulae (I), (II), (II') or (II") mentioned in one of claims 1 to 5.

**7.** Process for producing the complex compounds of formula (II), (II') or (II") mentioned in claims 2, 3 or 4, **charac-**

**terised in that** (BH) or (B'H) in the formulae $(BH)_{3-n-p-2pr}[RuX_{6-n-p-q-2r}B_n(H_2O)_p(OH)_q(O)_r]_{2r+1}$ or $(B'H)_{3-n-p-2pr}[RuX_{6-n-p-q-2r}B'_n(H_2O)_p(OH)_q(O)_r]_{2r+r}$ are replaced by M, M' or M'', wherein B, B', M, M', M'' have the meanings indicated in claims 2, 3 or 4.

8. Sterile aqueous solutions containing one or more of the complex compounds of formulae (I), (II), (II') or (II'') mentioned in one of claims 1 to 5.

9. Use of one of the complex compounds of formulae (I), (II), (II') or (II'') mentioned in one of claims 1 to 5 for producing medicament formulations for treating cancers.

**Revendications**

1. Préparations médicamenteuses, contenant un ou plusieurs composés complexes de formule générale I

$$\{G\}^{-(n+p+2r(p-1)-3)}\{[RuX_{6-n-p-q-2r}B_n(H_2O)_p(OH)_q(O)_r]_{2r+1}\}^{n+p+2r(p-1)-3} \quad (I)$$

dans laquelle

$$n+p+2r(p-1)-3q \neq 0$$

G est un contre-ion approprié, mais n'est pas un hétérocycle chargé,
B est un hétérocycle basique mono- ou polynucléaire, avec un ou plusieurs atomes d'azote,
X représente des halogénures, des pseudo-halogénures, $HCO_3^-$, $RCOO^-$ avec R = alkyle, alcényle ayant 1 à 6 atomes de carbone (substitué et non substitué), aryle (substitué et non substitué),
n est 1, 2 ou 3, mais $n + p \neq 3$
p est 0 ou 1, ou encore 0 ou 0,5 p si r = 0,5
q est 0 ou 1, ou encore 0 ou 0,5 q si r = 0,5, $\Sigma$ n, p, q $\leq$ 6, et
r est 0 ou 0,5.

2. Préparations médicamenteuses contenant un ou plusieurs composés complexes de formule générale II

$$M_{3-n-p-2pr}[RuX_{6-n-p-q-2r}B_n(H_2O)_p(OH)_q(O)_r]_{2r+1} \quad (II)$$

dans laquelle

M est un cation d'un métal alcalin ou ammonium,
B est un hétérocycle basique mono- ou polynucléaire ayant un ou plusieurs atomes d'azote,
X est un halogène, de préférence chlorure ou bromure,
n est 1 ou 2,
p est 0 ou 1, ou encore 0 ou 0,5 si r = 0,5,
q est 0 ou 1, ou encore 0 ou 0,5 si r = 0,5, et
r est 0 ou 0,5.

3. Préparations médicamenteuses contenant un ou plusieurs composés complexes de formule générale II'

$$M'_{3-n-p-2pr}[RuX_{6-n-p-q-2r}B'_n(H_2O)_p(OH)_q(O)_r]_{2r+r} \quad (II')$$

M' est lithium, sodium ou potassium,
B' est 1H-indazole ou 1H-imidazole et les dérivés de ceux-ci,
X est chlore,
p est 0 ou 1, ou encore 0 ou 0,5 si r = 0,5, mais 0 quand $q \neq 0$
q est 0 ou 1, ou encore 0 ou 0,5 si r = 0,5, mais 0 quand $p \neq 0$,

n et r ont la signification ci-dessus.

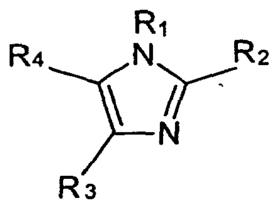

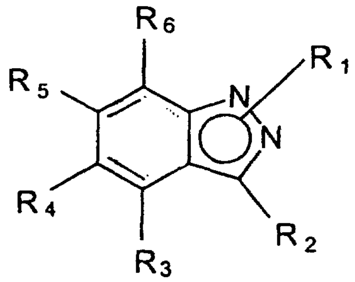

$R^1...R^6$ = H, alkyle, alcényle et aryle, ayant 1 à 6 atomes de carbone, substitués et non substitués.

**4.** Préparations médicamenteuses contenant un ou plusieurs composés complexes de formule générale II"

$$M''_{3-n-p-2pr}[RuX_{6-n-p-q-2r}B'_n(H_2O)_p(OH)_q(O)_r]_{2r+r} \quad (II'')$$

dans laquelle

M" est sodium, B', X, n, p, q et r ont la signification ci-dessus.

**5.** Préparations médicamenteuses contenant du transtétrachlorobis(imidazole)ruthénate(III) de sodium ou du trans-tétrachlorobis(indazole)ruthénate(III) de sodium.

**6.** Préparations médicamenteuses comprenant un excipient acceptable d'un point de vue pharmaceutique et 0,1 à 99,5 % en poids d'au moins un des composés complexes de formule I, II, II' ou II" mentionnés dans l'une des revendications 1 à 5.

**7.** Procédé de préparation des composés complexes de formule II, II' ou II" mentionnés dans la revendication 2, 3 ou 4, **caractérisé en ce que** (BH) et (B'H) dans les formules $(BH)_{3-n-p-2pr}[RuX_{6-n-p-q-2r}B_n(H_2O)_p(OH)_q(O)_r]_{2r+1}$ et $(B'H)_{3-n-p-2pr}[RuX_{6-n-p-q-2r}B'_n(H_2O)_p(OH)_q(O)_r]_{2r+r}$ sont remplacés respectivement par M, M' et M", B, B', M, M', M" ayant les significations indiquées dans la revendication 2, 3 ou 4.

**8.** Solutions aqueuses stériles contenant un ou plusieurs des composés complexes de formule I, II, II' ou II" mentionnés dans l'une des revendications 1 à 5.

**9.** Utilisation de l'un des composés complexes de formule I, II, II' ou II" mentionnés dans l'une des revendications 1 à 5, pour fabriquer des préparations médicamenteuses pour le traitement de maladies cancéreuses.